(19) Europäisches Patentamt
European Patent Office
Office européen des brevets

(11) Veröffentlichungsnummer: **0 207 287**
**A2**

# (12) EUROPÄISCHE PATENTANMELDUNG

(21) Anmeldenummer: 86107156.1

(22) Anmeldetag: 27.05.86

(51) Int. Cl.⁴: C 07 D 209/08
C 07 D 215/06, A 61 K 7/40

(30) Priorität: 04.06.85 DE 3519926

(43) Veröffentlichungstag der Anmeldung: 07.01.87 Patentblatt 87/2

(84) Benannte Vertragsstaaten: AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Scholl, Thomas, Dr.
Breslauer Strasse 31
D-4150 Krefeld 11(DE)

(72) Erfinder: Exner, Otto, Dr.
Richard-Wagner-Strasse 1
D-4019 Monheim(DE)

(72) Erfinder: Finkel, Peter, Dipl.-Ing.
Isidor-Caro-Strasse 56
D-5000 Köln 80(DE)

(72) Erfinder: Perrey, Hermann, Dr.
Auf der Rheinaue 8
D-4150 Krefeld 11(DE)

## (54) Indolinderivate, Verfahren zu ihrer Herstellung und diese Indolinderivate enthaltende kosmetische Lichtschutzmittel.

(57) Neue Indolinderivative der allgemeinen Formel

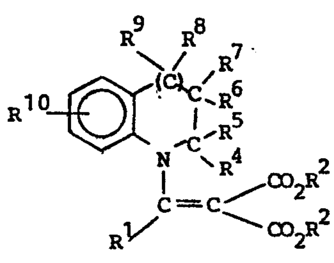

in welcher $R^1$ bis $R^{10}$ die in der Beschreibung angegebenen Bedeutungen haben, sind UV-A-Absorber mit hohen molaren Extinktionskoeffizienten (E) und Absorptionsmaxima im Bereich von 330 bis 345 nm. Sie werden nach an sich bekannten Verfahren hergestellt.

Die neuen Verbindungen sollen als Wirkstoffe in Lichtschutzmitteln verwendet werden.

EP 0 207 287 A2

BAYER AKTIENGESELLSCHAFT 5090 Leverkusen, Bayerwerk
Konzernverwaltung RP
Patentabteilung Si/ABc

Indolinderivate, Verfahren zu ihrer Herstellung und diese Indolinderivate enthaltende kosmetische Lichtschutzmittel

Die vorliegende Erfindung betrifft neue Indolinderivate, die sich als UV-Absorber eignen, und Kompositionen, die diese UV-Absorber zum Schutz der Haut von UV-Strahlung enthalten.

Die Sonnenstrahlung umfaßt unter anderem den Bereich der ultravioletten Strahlung, der im Hinblick auf die Wirkung auf die menschliche Haut in verschiedene Bereiche aufgeteilt werden kann:

a) UV-Strahlung des Wellenlängenbereiches von 285-320 nm (UV-B-Bereich) verursacht bei normalhäutigen Menschen einen Sonnenbrand (Erythem) und aufgrund einer photoinduzierten Melanogenese anschließende Pigmentation (indirekte Pigmentierung).

b) UV-Strahlung des Wellenlängenbereiches von 320-400 nm (UV-A-Bereich) bewirkt eine rasche, schwach ausgebildete Braunfärbung (Direktpigmentierung). Dieser Pigmentation liegt eine Photooxidation bestimmt ., in der Haut vorhandener Vorläufer des Melamins zugrunde.

Die üblichen Sonnenschutzmittel enthalten UV-B-Absorber, die die UV-B-Strahlung mehr oder weniger stark absorbieren. Für besonders lichtempfindliche Personen und um bei intensiverer Sonnenstrahlung die gesamte Belastung der Haut durch UV-Strahlung auf ein gesundes Maß zu beschränken, sind jedoch Sonnenschutzmittel erforderlich, die auch UV-A-Strahlung absorbierende Stoffe (UV-A-Absorber) enthalten.

Über den Einsatz in kosmetischen Sonnenschutzmitteln hinaus sind UV-A-Filter in der Dermatologie für solche Präparate von Interesse, die zur Chemotherapie von chronischen Lichtschäden, der Psoriasis und von gewerblichen Fotodermatosen, wie sie beim Umgang mit Teer, Kohle und Pech auftreten können, eingesetzt werden können.

Darüber hinaus bestehen Zusammenhänge zwischen der Belastung der Haut durch UV-A-Strahlung und ihrer Alterung. Außerdem gilt es als gesichert, daß eine größere Belastung der Haut durch UV-A-Strahlung, insbesondere bei genetisch bedingter Sensibilität, ein größeres Risiko an Hautkrebs zu erkranken bedeutet. Aus diesen Gründen sind UV-A-Absorber für kosmetische und medizinische Zwecke von besonderer Bedeutung.

Anforderungsschwerpunkte, die an solche kosmetischen UV-Absorber gestellt werden, sind nicht wie bei UV-Absorbern für Kunststoffe extreme UV- und Thermostabilität, sondern insbesondere:

1. eine hohe spezifische Extinktion $E_1$ (das bedeutet: Wirtschaftlichkeit, geringere Belastung der Rezeptur und geringeres toxikologisches Risiko, da weniger auf die Haut gelangt),

2. eine ausgezeichnete Öllöslichkeit (kein Ausfallen bei tiefen Temperaturen, z.B. beim Transport oder Verwendung beim Wintersport),

3. pH-Stabilität,

4. toxikologische Unbedenklichkeit,

5. keine deutliche Eigenfärbung.

UV-A-Absorber für kosmetische Zwecke sind zwar prinzipiell nicht neu. Die bekannten UV-A-Absorber erfüllen jedoch die oben genannten Bedingungen nicht in ausreichendem Maße, insbesondere nicht, was das Absorbtionsmaximum, die Höhe der spezifischen Extinktion $E^1$ und die Öllöslichkeit abbetrifft.

Gegenstand der Erfindung sind neue Indolinderivate, die sich als kosmetische UV-Absorber eignen und die den genannten Anforderungen in idealer Weise entsprechen. Sie werden durch die nachstehende Formel I wiedergegeben:

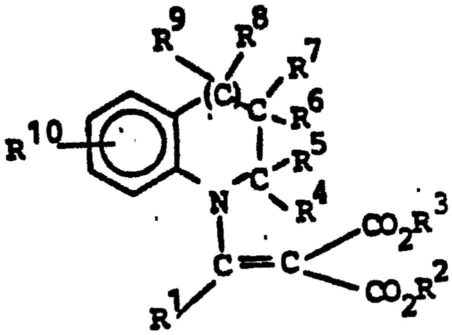

(I)

In Formel I bedeuten

$R^1$ H oder $C_1$-$C_8$-Alkyl:

$R^2$ und $R^3$ gleiches oder verschiedenes $C_1$-$C_8$-Alkyl:

$R^4$ - $R^9$ gleiches oder verschiedenes H, $C_1$-$C_8$-Alkyl, $C_5$-$C_6$-Cycloalkyl oder Phenyl-$C_1$-$C_4$-alkyl, wobei $R^5$ und $R^6$ in dem Sinne miteinander verbunden sein können, daß ein weiterer $C_5$-$C_6$-Ring vorliegt, und

$R^{10}$ H oder einen oder mehrere $C_1$-$C_8$-Alkyl, $C_1$-$C_8$-Alkoxy-, $C_5$-$C_6$-Cycloalkyl-, Phenyl-$C_1$-$C_4$-alkyl-, Carb-$C_1$-$C_8$-alkoxyreste oder Halogenatome.

Die neuen Verbindungen unterscheiden sich von den bekannten offenkettigen Verbindungen der Formel II

II

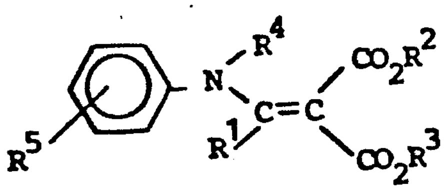

$R^1$-$R^4$= H, Alkyl, Aryl

$R^5$ = H, Alkyl, Halogen, Carboxyalkyl, Aryl

wie sie in der US-Patentschrift 3 079 366 beschrieben sind, dadurch, daß sie den Stickstoff als Bestandteil eines am Phenylkern kondensierten Ringes enthalten. Dieser Unterschied bewirkt in nicht voraussehbarer Weise, daß die neuen cyclischen Verbindungen der Formel I erheblich lichtechter als die bekannten Verbindungen gemäß US 3 079 366 sind. Gerade der Lichtechtheit kommt aber eine außerordentliche Bedeutung für die erfolgreichen Verwendungen als UV-Absorber zu.

Aus der DE-OS 3 018 132 sind antibakterielle Hautpflegemittel IV bekannt, unter anderem der Formel IV a

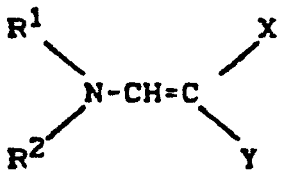

IV

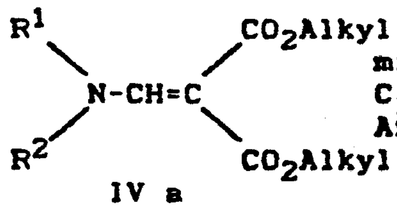

IV a

mit $R^1$, $R^2$ = H, $C_1$-$C_{14}$-Alkyl, Aryl, Aralkyl

X, Y = $CO_2$Alkyl, CN

Die lichtschützende Wirkung dieser Verbindungen und insbesondere der Einfluß des heterocyclisch gebundenen Stickstoffs auf die Absorptionslage und die UV-Stabilität wurde jedoch nicht erkannt. Die überraschende Feststellung der vorliegenden Erfindung ist zudem das völlig gegensätzliche galenische Verhalten von Aminomethylen-malonsäureestern und Aminomethylen-cyanessigestern, bei einer als ideal zu bezeichnenden UV-A-Absorptionslage. Hinzu kommt, daß die

Wirkungskonzentration der antibakteriellen Hautpflegemittel gemäß DE-OS 3 018 132 wesentlich (vorzugsweise 3-15 %) über der Wirkungskonzentration der erfindungsgemäßen UV-A-Absorber liegt (vorzugsweise 0,1-3 %).

Da nahezu die gesamte Klasse der erfindungsgemäßen UV-A-Absorber ölig ist, wird der Fachmann der Verbindung mit dem kleinsten Molekulargewicht den Vorzug geben, da hier das Absorptions/Gewichts-Verhältnis am besten ist.

Die erfindungsgemäßen Verbindungen besitzen einen hohen molaren Extinktionskoeffizienten (E) und ihre Absorptionsmaxima liegen im Bereich von 330-345 nm. Die Absorptionsmaxima sind im Vergleich zu den offenkettigen Verbindungen der US-PS 3 079 366 deutlich langwelliger und verleihen erst hierdurch den erfindungsgemäßen Verbindungen der Formel I ihre Wirksamkeit als UV-A-Absorber. Die erfindungsgemäßen Verbindungen zeigen einen so steilen Abfall zum sichtbaren Bereich, daß sie trotz ihrer langwelligen Absorption nur sehr schwach gelblich gefärbt sind.

Im Gegensatz zu den Verbindungen der DE-PS 1 568 341 der allgemeinen Formel III,

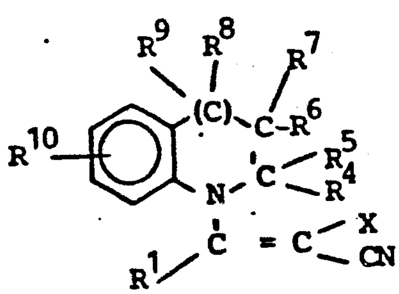

III

mit X = CN, $CO_2$Alkyl,
R = gemäß Bedeutung der Formel I

die allesamt kristallin sind und schwer löslich in unpolaren Lösungsmitteln, sind die erfindungsgemäßen Verbindungen fast ausnahmslos ölig und zeichnen sich, nicht zuletzt aus diesem Grund, durch eine überragende Löslichkeit in allen gängigen kosmetischen Rezepturen aus.

Beispiele für die erfindungsgemäßen Verbindungen sind:

Tabelle 1

| | | $\lambda_{max}$ | E |
|---|---|---|---|
| 1 | 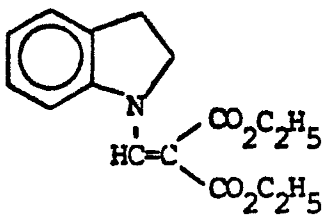 | 330 nm | (32.196) |
| 2 | 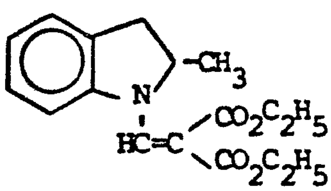 | 330 nm | (30.025) |
| 3 | 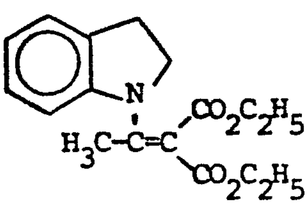 | 338 nm | |
| 4 | 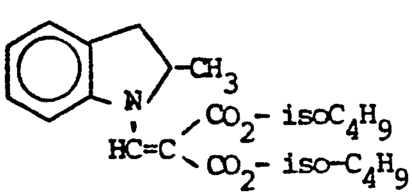 | 331 nm | (26.233) |

<u>Tabelle 1</u> (Fortsetzung)

| | | $\lambda_{max}$ | E |
|---|---|---|---|
| 5 | 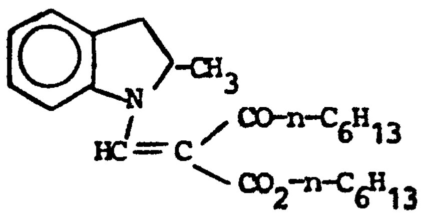 | 332 nm | (25.310) |
| 6 | 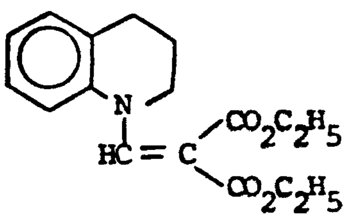 | 317 nm | (26.680) |

Die erfindungsgemäßen UV-A-Absorber können nach an sich bekannten Verfahren hergestellt werden, z.B. durch Umsetzung von Malonsäureestern (VI) mit Orthocarbonsäureestern (V), wie Orthoameisensäure-trimethylester, Orthoameisensäuretriethylester, Orthoessigsäure- trimethylester, Orthoessigsäure-triethylester, Orthopropionsäure-triethylester etc. gemäß Schema A:

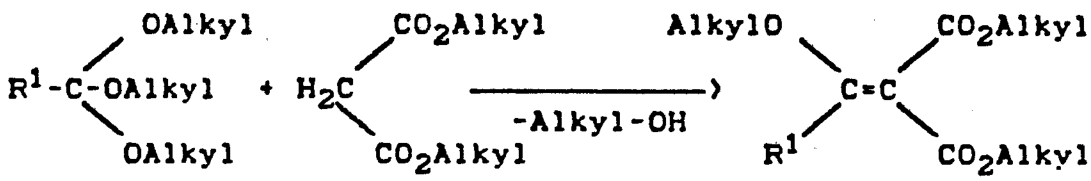


zu den entsprechenden Alkoxyalkylenmalonsäureestern (VII) und anschließende Kondensation nach Schema B mit NH-Heterocyclen (VIII), wie Indolin, 2-Methylindolin, 2.3.3-Trimethyl-5-methoxyindolin, 1.2.3.4.10.11-Hexahydrocarbazol, 1.2.3.4-Tetrahydrochinolin und 1.2.3.4-Tetrahydrochinaldin,

B:

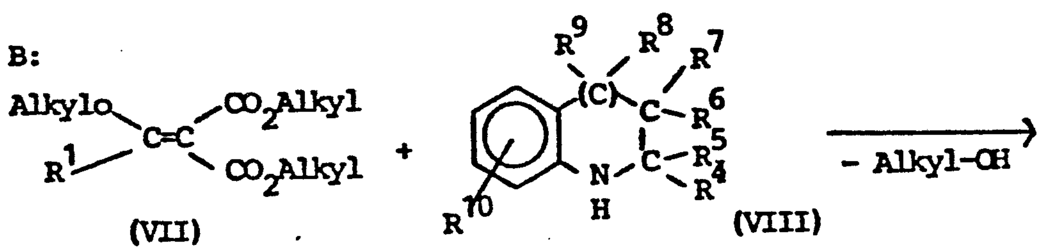


(VII) (VIII)

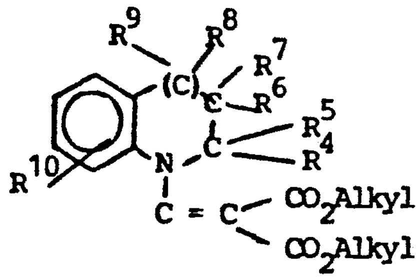

(I)

wobei die Reste $R^1$-$R^{10}$ die für die Formel I genannte Bedeutung haben.

Reaktionen gemäß Schema A sind bekannt und z.B. in Org. Synthesis Coll. Vol. 3, 395 (1955), beschrieben. Sie werden gewöhnlich in wasserentziehenden Lösungsmitteln durchgeführt, wie z.B. Acetanhydrid und in Gegenwart katalytischer Mengen Lewissäuren wie z.B. Zinkchlorid. Reaktionen gemäß Schema B sind ebenfalls bekannt und z.B. in US 3 079 366 beschrieben. Sie werden durch gemeinsames Erhitzes der beiden Komponenten auf Temperaturen zwischen 100-200°C und Abdestillieren des Alkanols durchgeführt.

Eine andere Herstellungsvariante stellt das gemeinsame Erhitzen von Orthocarbonsäureestern, Malonsäureestern und NH-Heterocyclen dar, wie durch Schema C beschrieben wird.

C: 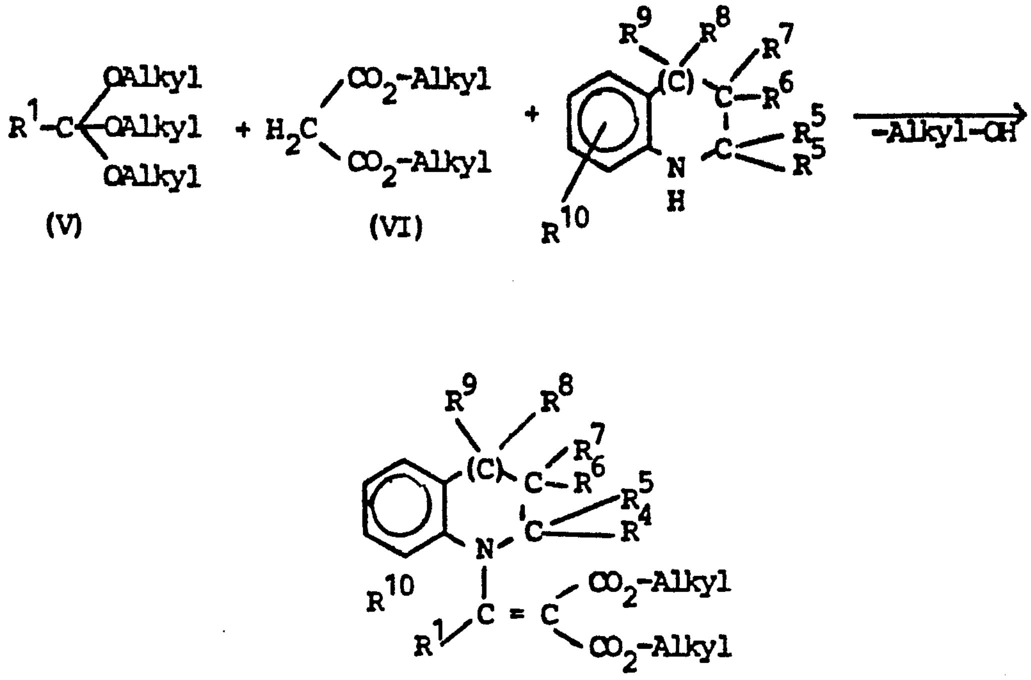


(V) (VI)

(I)

Derartige Verfahren sind ebenfalls bekannt und in US 3 079 366 beschrieben. Die Reaktionstemperaturen liegen bevorzugt zwischen 100 und 200°C.

UV-Absorber für kosmetische und dermatologische Lichtschutzmittel sind nicht neu. So werden z.B. 2.2'-Dihydroxy-4,4'-dimethoxy-benzophenon-5-sulfonsäure-Natriumsalz (Vergleichbeispiel 1), 2,2',4,4'-Tetrahydroxy-benzophenon (Vergleichsbeispiel 2) und Dianisoylmethan (Vergleichsbeispiel 3) auf dem Markt angeboten. Die Eigenschaften dieser bekannten Verbindungen werden denen der erfindungsgemäßen UV-A-Absorber der Formel I in Tabelle 2 gegenübergestellt.

**Tabelle 2**

| | | $\lambda_{max}$ (nm) | spez. Extinktion $E^1$ | Löslichkeit bei RT Erdnußöl | Ernuß/Paraffinöl = 1:9 |
|---|---|---|---|---|---|
| 1) | 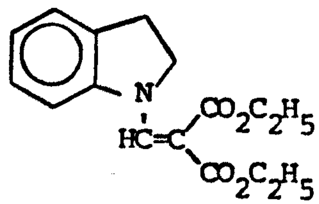 | 330 | 1114 | ∞ | ∞ |
| 2) | 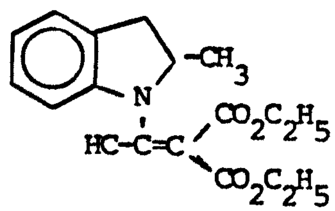 | 330 | 1008 | ∞ | ∞ |
| 3) | 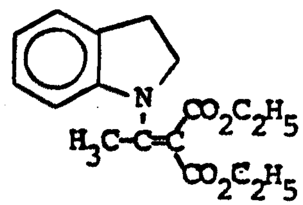 | 338 | | ∞ | ∞ |
| 4) | 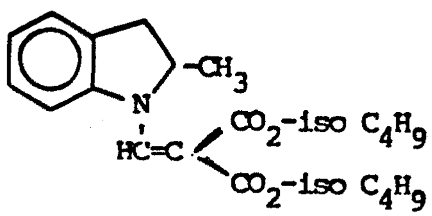 | 331 | 730 | ∞ | ∞ |

Le A 23 848

**Tabelle 2**

| | | $\lambda_{max}$ (nm) | spez. Extinktion $E^1$ | Löslichkeit bei RT Erdnußöl | Ernuß/Paraffinöl = 1:9 |
|---|---|---|---|---|---|
| 5) | 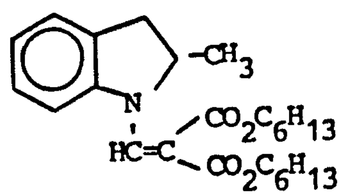 | 332 | 582 | ∞ | ∞ |
| 6) | 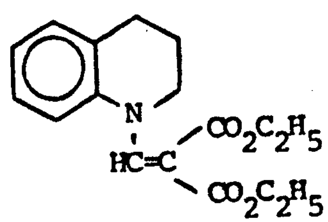 | 317 | 878 | ∞ | ∞ |
| | Vergleichsbeispiel 1 | 330 | 160 | - | - |
| | Vergleichsbeispiel 2 | 351 | 645 | 0,8 % | - |
| | Vergleichsbeispiel 3 | 362 | 1270 | 1 % | - |

Aus diesem Vergleich gehen die überlegenen Eigenschaften der erfindungsgemäßen Verbindungen hervor. Die bekannten Verbindungen sind von der Löslichkeit her ungeeignet. Vergleichsbeispiele (1) und (2) haben eine deutlich geringere spezifische Extinktion. Vergleichsbeispiel 3 hingegen hat zwar eine hohe spezifische Extinktion, die aber zu langwellig ist, so daß der wichtige Bereich zwischen 320-340 nm nicht voll abgedeckt ist.

Gegenstand der Erfindung sind auch Lichtschutzmittel, die in einer Ölphase mindestens eine der erfindungsgemäßen Verbindungen der Formel I als UV-A-Absorber zum Schutz der Haut gegen UV-A-Strahlung neben anderen geeigneten kosmetischen oder dermatologischen Zusätzen enthalten. Diese Lichtschutzmittel können zum Beispiel Anwendung finden als Schutz von natürlicher Sonnenstrahlung, z.B. bei Freizeit oder gewerblich bedingtem Aufenthalt im Sonnenlicht, vor künstlichen Lichtquellen, z.B. Höhensonne und Solarium und zur Behandlung von pathogenen Sensibilitäten, z.B. genetische oder gewerbliche, gegenüber UV-A-Strahlung.

Insbesondere sind folgende öllösliche UV-A-Filter für den erläuterten Verwendungszweck hervorragend geeignet:

N-(Dicarboethoxy-vinyl)-indolin (1)
N-(Dicarboethoxy-vinyl)-2-methylindolin (2)
N-(α-Methyl-β,β-dicarboethoxy-vinyl)-indolin (3)
N-(Dicarbo-iso-butoxy-vinyl)-2-methylindolin (4)
N-(Dicarbo-hexyloxy-vinyl)-2-methyl-indolin (5)
N-Dicarboethoxy-vinyl)-1,2,3,4-tetrahydrochinolin (6)

Besonders bevorzugt ist die Verbindung (2): N-(Dicarboethoxy-vinyl)-2-methylindolin.

Die erfindungsgemäßen Lichtschutzmittel können in Form einer Flüssigkeit vorliegen, die aus einer einzigen Phase besteht und die ein einziges Lösungsmittel oder eine Mischung von Lösungsmitteln, z.B. eine Oleo-Alkohol-Mischung enthält. Sie können auch als Dispersion, als homogene Paste, als halbfests Produkt oder als Produkt, das ein Treibmittel enthält, vorliegen. Sie können Sonnenschutzmittel bilden, wie Öle, Lotionen, Aerosole (vom Typ Öl, Schaum, Spray) sowie auch Creme für normale oder trockene Haut, Milch, Lippenstifte oder jegliche andere übliche kosmetische oder dermatologische Zubereitung.

Als Bestandteile für die erfindungsgemäßen, oben näher definierten Mittel kann man insbesondere nennen: Lanolin, Vaseline, Triglyceride von Fettsäuren, Polyethylenglykole, ethoxylierte Fettalkohole, Ester wie Isopropylpalmitat, Isopropylmyristat, Isopropylstearat, Oleyloleat, Butylstearat, tierische, pflanzliche, synthetische oder mineralische Öle, Fettalkohole, niedere Alkohole, organische und mineralische Wachse. Diese Bestandteile werden in Mengen von etwa 1-97 Gew.-% verwendet.

Die erfindungsgemäßen Verbindungen der Formel I werden in den Lichtschutzmitteln gemäß der Erfindung in einer Konzentration von 0,2 bis 10 Gew.-%, bezogen auf das Gewicht der Zubereitung und vorzugsweise in 0,5 bis 6 % verwendet, wobei der Rest der Zubereitung auf 100 Gew.-% ergänzt wird

durch einerseits die üblichen kosmetischen bzw. dermatologischen Ingedienzen und andererseits das Lösungsmittel oder eine Mischung der Lösungsmittel.

Unter den kosmetischen und dermatologischen Zusätzen kann man Eindickungsmittel, reizlindernde und entzündungshemmende Mittel, Überfettungsmittel, Weichmacher, Benetzungsmittel, oberflächenaktive Mittel sowie Konservierungsmittel, Antischaummittel, Parfüms bzw. Riechstoffe oder jedem anderen brauchbaren Zusatz nennen, wie er in der Kosmetik oder der Dermatologie für den angestrebten Verwendungszweck üblich ist.

Die erfindungsgemäßen Mittel können farblos sein oder gefärbt sein mit solchen Farbstoffen und/oder Pigmenten, die gewöhnlich für Sonnenschutzmittel verwendet werden und insbesondere mit Eisenoxiden in Anteilen von etwa 0.001 bis 0,050 Gew.-%, bezogen auf das Gesamtgewicht der Zubereitung.

Enthält das erfindungsgemäße Mittel ein Treibmittel, so verwendet man insbesondere die Treibmittel auf Basis Chlorfluormethanen.

Die erfindungsgemäßen Zubereitungen können neben den UV-A-Filtern der Formel I auch UV-B-Filter enthalten, wie z.B.:

p-Dimethylaminobenzoesäure-hexylester
Salicylsäurehomomethylester

Anthranilsäuremethylester
p-Methoxyzimtsäure-isoamylester
α-Cyano-β-phenylzimtsäure-ethylester
2-Hydroxy-4-methoxy-benzophenon
3-(4-Methylbenzyliden)-D,L-campher
2-Phenylbenzimidazol-5-sulfonsäure
2-Phenyl-5-methyl-benzoxazol.

Die genannten Beispiele stehen nur repräsentativ für die jeweilige insgesamt gebräuchliche Verbindungsklasse. Weitere Beispiele finden sich in E. Charlet, P. Finkel, Ärztliche Kosmetologie 8, 302-311 (1978). Besonders geeignete UV-B-Absorber sind 2-Phenyl-5-methyl-benzoxazol, 2-Phenyl-benzimidazol-5-sulfonsäure und 4-Methoxy-zimtsäure-isoamylester.

Beispiel 1

N-(Dicarboethoxy-vinyl)-2-methylindolin

Ein Gemisch aus 216 g Ethoxymethylenmalonsäure-Ester und 131 g 2-Methylindon wird 5 h auf 150°C erhitzt, so daß 46 g Ethanol abdestillieren. Anschließend wird der Rückstand fraktioniert destilliert. Man erhält ca. 270 g gelbliches Öl vom $Kp_{0,3}$ 185°C.

Beispiele 2 - 6

Die folgenden Verbindungen können nach dem Verfahren des Beispiels 1 erhalten werden:

N-(Dicarboethoxy-vinyl)-indolin, $Kp_{0,2}$ 180°C (95 %)

N-(α-Methyl-β-β-dicarboethoxy-vinyl)-indolin, $Kp_{0,3}$ 178°C

N-(Dicarbo-iso-butoxy-vinyl)-2-methylindolin, $Kp_{0,4}$ 195°C (85 %)

N-(Dicarbo-hexyloxy-vinyl)-2-methylindolin, $Kp_{0,65}$ 245°C (80 %)

N-(Dicarboethoxy-vinyl)-1.2.3.4-tetrahydrochinolin $Kp_{0,3}$ 184°C (95 %)

**Beispiel 7:** Sonnenschutzöl

| | |
|---|---|
| N-(Dicarboethoxy-vinyl)-2-methylindolin | 3 % |
| Paraffinöl | 37 % |
| Isopropylpalmitat | 60 % |
| Parfümöl | q.s. |

**Beispiel 8:** Sonnenschutzöl

| | |
|---|---|
| N-(Dicarboethoxy-vinyl)-2-methylindolin | 2 % |
| p-Methoxy-zimtsäure-isoamylester | 2 % |
| Erdnußöl | 46 % |
| Paraffinöl | 50 % |
| Parfümöl | q.s. |

Die Herstellung der Sonnenschutzöle erfolgt durch Mischen der angeführten Komponenten.

**Beispiel 9:** Lippenstifte

| | |
|---|---|
| Handelsübliche Lippenstiftmasse | 94 % |
| N-(Dicarboethoxy-vinyl)-2-methylindolin | 3 % |
| p-Methoxyzimtsäure-isoamylester | 3 % |

Die Lippenstiftmasse wird aufgeschmolzen und mit den beiden übrigen Komponenten gemischt. Die Masse wird in gekühlten Formen vergossen und die Formkörper nach dem Erkalten entnommen.

0207287

**Beispiel 10:** Sonnenschutz-Spray

| | |
|---|---|
| Mischung nach Beispiel 7 | 40 % |
| Treibgas-Mischung von Trifluorchlormethan und Dichlorfluormethan 70:50 | 60 % |

Die beiden Komponenten werden in einem entsprechenden Druckgasbehälter gefüllt.

**Beispiel 11:** Sonnenschutzcreme Typ O/W

| | |
|---|---|
| A - Fettalkohol-polyglykolether auf der Basis von Stearyl-n-cetylalkohol | |
| - Entenburzeldrüsenfett (künstlich) | 5.0 % |
| - Isopropylpalmitat | 6.0 % |
| - Caprylcaprinsäure-triglycerid | 11.0 % |
| - Cetylstearylalkohol | 2.0 % |
| - Silikonöl cp 100 | 0,5 % |
| - N-(Dicarboethoxy-vinyl)-2-methylindolin | 2.0 % |
| B - Wasser, entmineralisiert | 66 % |
| - Parfümöl | q.s. |
| - Konservierungsmittel | q.s |

Die Komponenten werden bis 70°C aufgeschmolzen und gemischt. Unter Rühren wird die Mischung portionsweise in das auf 75°C erwärmte Wasser gegeben. Die entstandene Emulsion wird langsam und unter weiteren Rühren auf Raumtemperatur abgekühlt.

**Beispiel 12:** Sonnenschutz-Milch

| | | |
|---|---|---|
| A | - Kolloiddisperses Gemisch von Cetylstearylalkohol und Natrium-cetylstearylsulfat mit einem nicht ionogenen Emulgator | 3.15 % |
| | - Decyloleat | 15.00 % |
| | - N-(Dicarboethoxy-vinyl)-2-methylindolin | 2.00 % |
| B | - Wasser | 79.85 % |
| | - Parfüm | q.s. |
| | - Konservierungsmittel | q.s. |

Herstellung wie Beispiel 11.

Patentansprüche

1. Indolinderivate der Formel I

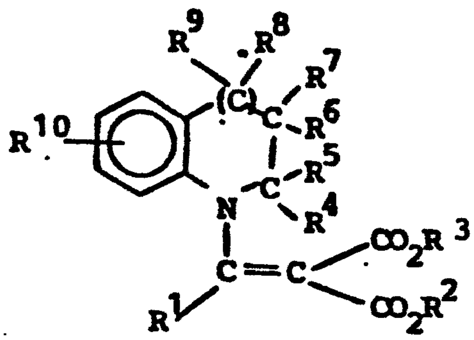

(I)

in der

$R^1$ H oder $C_1$-$C_8$-Alkyl bedeutet,

$R^2$ und $R^3$ gleiches oder verschiedenes $C_1$-$C_8$-Alkyl bedeutet,

$R^4$ bis $R^9$ gleich oder verschieden sind und H, $C_1$-$C_8$-Alkyl, $C_5$-$C_6$-Cycloalkyl oder Phenyl-$C_1$-$C_4$-alkyl, wobei $R^5$ und $R^6$ verbunden sein können,

$R^{10}$ H oder einen oder mehrere $C_1$-$C_8$-Alkyl-, $C_1$-$C_8$-Alkoxy-, $C_5$-$C_6$-Cycloalkyl-, Phenyl-$C_1$-$C_4$-alkyl-, Carb-$C_1$-$C_8$-alkoxyester oder Halogenatome,

bedeuten.

2. N-(Dicarbethoxy-vinyl)-2-methylindolin.

3. Verfahren zur Herstellung von Indolinderivaten der Formel I in Anspruch 1, dadurch gekennzeichnet,

daß man entweder

a) Malonsäureester (VI) mit Orthocarbonsäureestern (V) umsetzt zu Alkoxyalkylenmalonsäureestern (VII) und diese mit NH-Heterocyclen kondensiert:

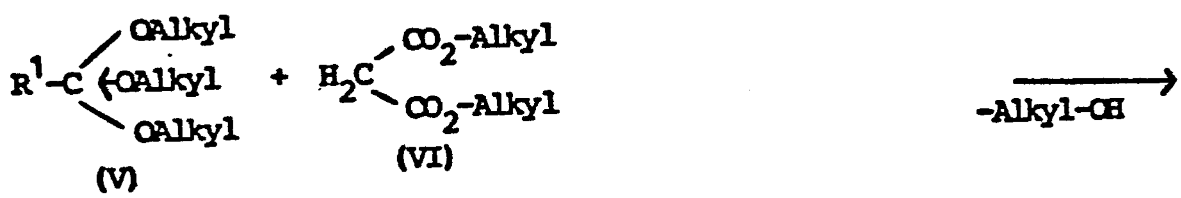


(V) (VI)

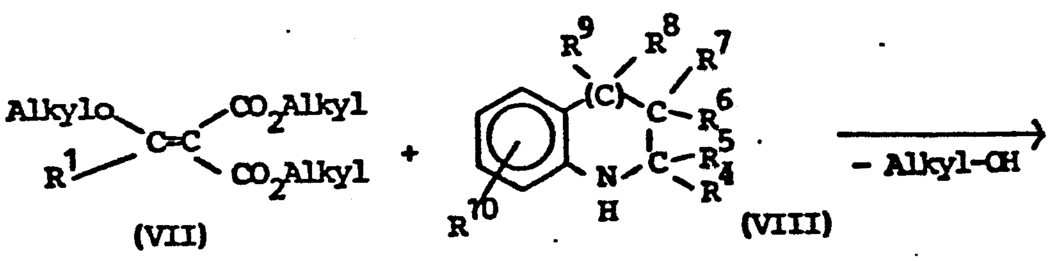


(VII) (VIII)

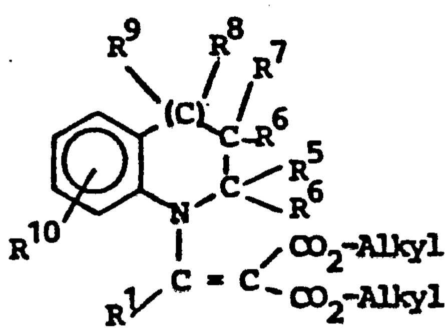

(I)

oder daß man

b) Malonester (VI), Orthocarbonsäureester (V) und NH-Heterocyclen (VIII) zusammen erhitzt:

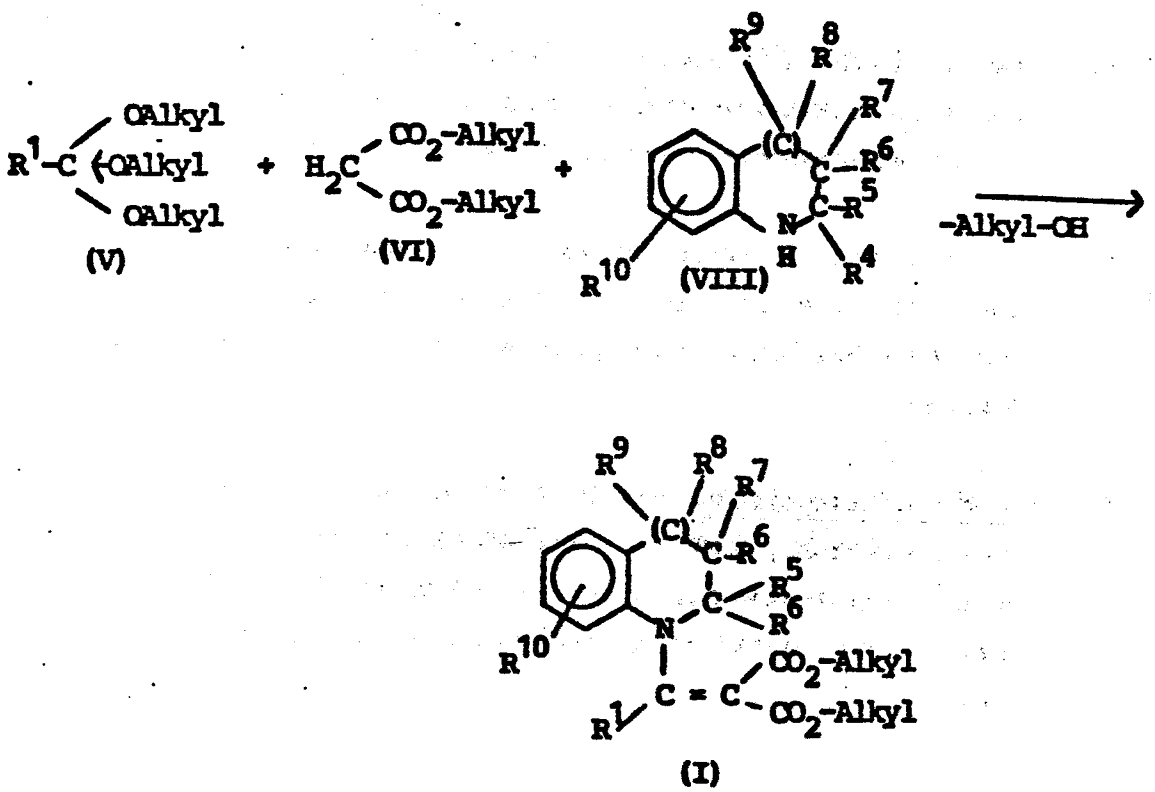


(V) (VI) (VIII) (I)

4. Verwendung der Indolinderivate der Formel I in Anspruch 1 als UV-A-Absorber in kosmetischen Lichtschutzmitteln.

5. Kosmetische Lichtschutzmittel, dadurch gekennzeichnet, daß sie mindestens einen UV-A-Absorber der Formel I in Anspruch 1 in Konzentrationen von 0,1-10 Gew.-% enthalten.

6. Kosmetische Lichtschutzmittel, dadurch gekennzeichnet, daß sie neben 0,1-10 Gew.-% UV-A-Absorber der Formel I in Anspruch 1 zusätzlich 0,1-10 Gew.-% üblicher UV-B-Absorber enthalten.

7. Kosmetische Lichtschutzmittel gemäß Anspruch 1, dadurch gekennzeichnet, daß der UV-A-Absorber N-(Dicarboethoxy-vinyl)-2-methylindolin ist.

8. Kosmetische Lichtschutzmittel gemäß Anspruch 1, dadurch gekennzeichnet, daß sie bei Lichtschutzmitteln übliche Lösungsmitel und Zusatzstoffe enthalten.

9. Lichtschutzmittel gemäß Ansprüche 5-8, dadurch gekennzeichnet, daß sie als UV-B-Absorber 2-Phenyl-5-methyl-benzoxazol, 2-Phenyl-benzimidazol-5-sulfonsäure oder 4-Methoxy-zimitsäure-isoamylester enthalten.